# EUROPÄISCHE PATENTSCHRIFT

(11) **EP 1 944 284 B1**
(45) Veröffentlichungstag und Bekanntmachung des Hinweises auf die Patenterteilung: **29.06.2011**
(21) Anmeldenummer: 08000201.7
(22) Anmeldetag: 08.01.2008
(51) Int. Cl.: C07C 37/20, C07C 37/84

(54) **Kristallisationsverfahren zur Herstellung von Bisphenol A**
Crystallisation method for creating bisphenol A
Procédé de cristallisation destiné à la fabrication de bisphénol A

(30) Priorität: 09.01.2007 DE 102007001427
(43) Veröffentlichungstag der Anmeldung: 16.07.2008
(73) Patentinhaber: Bayer MaterialScience AG, 51368 Leverkusen (DE)
(72) Erfinder: Münnich, Christian, Dr., 51377 Leverkusen (DE); Blaschke, Ulrich, Dr., 47829 Krefeld (DE); Eek, Rob, Dr., Hong Kong (CN); Prein, Michael, Dr., 47809 Krefeld (DE); Audenaert, Raymond, Dr., 9220 Hamme (BE); Tytgat, Gert, 2570 Duffel (BE)

(56) Entgegenhaltungen:
- EP-A- 0 671 377
- EP-A- 1 728 777

## Beschreibung

Die vorliegende Anmeldung betrifft ein Verfahren zur Herstellung von Bisphenol A (BPA) mit einer Reinheit von größer 99,7 %.

Bisphenole als Kondensationsprodukte von Phenolen und Carbonylverbindungen sind Ausgangsstoffe oder Zwischenprodukte zur Herstellung einer Vielzahl kommerzieller Produkte. Von besonderer technischer Bedeutung ist das Kondensationsprodukt aus der Reaktion zwischen Phenol und Aceton, 2,2-Bis(4-hydroxyphenyl)propan (Bisphenol A, BPA). BPA dient als Ausgangsstoff zur Herstellung verschiedenartiger polymerer Werkstoffe wie beispielsweise Polyarylate, Polyetherimide und modifizierter Phenol-Formaldehydharze. Bevorzugte Anwendungsgebiete liegen in der Herstellung von Epoxyharzen und Polycarbonaten.

Verfahren zur Synthese von Bisphenol A (BPA) mittels Ionenaustauscherkatalyse sind bekannt, z.B. aus US-A 4 391 997, US-A 4 400 555, US-A 4 590 303 oder EP-A 210 366. Es ist bekannt, zur großtechnischen Herstellung von BPA ein Gemisch aus Phenol und Aceton durch einen mit sulfonsauren Ionentauscherharzen auf Polystyrolbasis gefüllten Festbettreaktor zu leiten und anschließend der Aufarbeitung zuzuführen.

Bei der Umsetzung von Phenol mit Aceton in Gegenwart saurer Katalysatoren entsteht eine Produktmischung (Reaktionsmischung), die neben nicht umgesetztem Phenol und gegebenenfalls Aceton in erster Linie BPA und Wasser enthält. Daneben treten in geringen Mengen typische Nebenprodukte (Nebenkomponenten) der Kondensationsreaktion auf, so beispielsweise 2-(4-hydroxyphenyl)-2-(2-hydroxyphenyl)propan (o,p-BPA), substituierte Indane, Hydroxyphenyl-indanole, Hydroxyphenyl-chromane, substituierte Xanthene und höher kondensierte Verbindungen mit drei oder mehr Phenylringen im Molekülgerüst. Außerdem können sich durch Eigenkondensation des Acetons und Reaktion mit Verunreinigungen in den Rohstoffen weitere Nebenkomponenten wie Anisol, Mesityloxid, Mesitylen und Diacetonalkohol bilden.

Die genannten Nebenprodukte wie auch Wasser, Phenol und Aceton beeinträchtigen die Eignung von BPA zur Herstellung von Polymeren und müssen durch geeignete Verfahren abgetrennt werden. Insbesondere zur Herstellung von Polycarbonat werden hohe Reinheitsanforderungen an den Rohstoff BPA gestellt.

Eine Aufarbeitungs- und Reinigungsmethode von BPA erfolgt durch Abtrennung von BPA aus der Reaktionsmischung in Form eines etwa äquimolaren kristallinen Addukts mit Phenol durch Abkühlen der Reaktionsmischung unter Auskristallisieren des BPA/Phenol-Addukts als Suspensionskristallisation. Die BPA/Phenol-Adduktkristalle werden anschließend durch eine geeignete Apparatur zur Fest-Flüssigtrennung wie Drehfilter oder Zentrifugen von der Flüssigphase abgetrennt und der weiteren Reinigung zugeführt.

So erhaltene Adduktkristalle weisen typischerweise eine Reinheit von > 99 % BPA bezogen auf die Nebenkomponenten bei einem Phenolanteil von ca. 40 % auf. Durch Waschen mit geeigneten Lösungen, die typischerweise eine oder mehrere Komponenten aus der Gruppe Aceton, Wasser, Phenol, BPA und Nebenkomponenten enthalten, können die Adduktkristalle von oberflächlich anhaftenden Verunreinigungen befreit werden.

Die im Anschluss an die oben beschriebene Suspensionskristallisation der Reaktionslösung und Fest-Flüssig-Trennung erhaltenen BPA-Phenol-Adduktkristalle werden weitergehenden Reinigungsschritten zugeführt, die beispielsweise destillative, desorptive oder extraktive Methoden enthalten, wobei die Abtrennung von Phenol und gegebenenfalls die Verringerung der Konzentration an Nebenkomponenten in BPA erzielt wird.

Alternativ kann das Phenol auch durch Ausschmelzverfahren aus den BPA-Phenol-Adduktkristallen entfernt werden.

An die Gewinnung von BPA-Phenol-Adduktkristallen aus dem Produktgemisch werden hohe Anforderungen gestellt. Neben dem in Überschuss vorliegenden Phenol und ggf. nicht umgesetztem Aceton müssen dabei noch Wasser und eine Vielzahl Nebenprodukte der Bisphenol A-Herstellung wie beispielsweise 2-(4-Hydroxyphenyl)-2-(2-hydroxyphenyl)propan (o,p-BPA), substituierte Indane, Hydroxyphenyl-indanole, Hydroxyphenyl-chromane, Spirobisindane, substituierte Indenole, substituierte Xanthene und höher kondensierte Verbindungen mit drei oder mehr Phenylringen im Molekülgerüst abgetrennt werden.

So beschreibt beispielsweise EP 1 268 379 B1 ein Verfahren zur Herstellung von BPA-Phenol-Adduktkristallen durch Kristallisation. Dabei wird bevorzugt durch eine zweistufig Kristallisation, wobei das Produktgemisch zunächst auf einen Bereich von 50 bis 70 °C und anschließend auf 40 bis 50 °C abgekühlt wird, die Herstellung von BPA mit einer Reinheit von 99,5 % beschrieben.

Da die Anforderungen an die Reinheit von Bisphenol A immer weiter steigen, sind weitere Verfahrensverbesserungen erforderlich, um die steigenden Qualitätsanforderungen z.B. für die Herstellung von Polycarbonat zu gewährleisten.

Dies kann beispielsweise durch eine Erhöhung der Verweilzeit in den Kristallern ermöglicht werden. So ist z.B. aus EP 1 607 380 A1 bekannt, dass bei einer kurzen Verweilzeit von 0,5 h oder weniger in der Kristallisation und entsprechend schneller Kristallisation der Einschluss von Reaktionsgemisch bzw. der Einbau von Verunreinigungen in die BPA-Phenol-Adduktkristalle zu einer schlechteren Bisphenol A Qualität führt, so dass Verweilzeiten im Bereich 1 - 3 h bevorzugt werden.

Ein weiterer Nachteil der in EP 1 268 379 B1 beschriebenen Methode ist außerdem, dass bei einer solchen zweistufigen Kristallisation in den Umwälzkühlern der 1. Stufe der Kristallisation (Hochtemperaturstufe) Ablagerungen an den Oberflächen (Fouling) auftreten können. Um die Ablagerungen beispielsweise durch Aufheizen auf ca. 80 °C zu beseitigen, muss der Umwälzkühler in regelmäßigen Zeitabständen außer Betrieb genommen werden, um die Ablagerungen abzuschmelzen. In dieser Zeit muss die Produktion angehalten oder die Kristallisation von der 2. Kristallisationsstufe alleine übernommen werden. Dies führt zu Produktionsausfall und / oder verringerter Qualität des Produkts, die z.B. durch einen erhöhten Anteil von Nebenprodukten (Verunreinigungen) in BPA gekennzeichnet ist.

EP 1 728 777 A und EP 0 671 377 A offenbaren Verfahren zur Herstellung von hochreinem Bisphenol A, in denen in Serie und parallel geschaltete Kristallisationsverfahren offenbart werden.

Aufgabe des vorliegenden Verfahrens ist es daher, ein hochreines Bisphenol A mit einer Reinheit > 99,7% herzustellen bei gleichzeitiger Verlängerung der Zeitintervalle zwischen den einzelnen Abschmelzvorgängen der Umwälzkühler.

Diese Aufgabe wird gelöst durch ein Verfahren zur Herstellung von Bisphenol A mit einer Reinheit von mehr als 99,7%, bei dem
a) Phenol und Aceton in Gegenwart eines sauren Katalysators zu einem Bisphenol A enthaltenden Produktgemisch umgesetzt werden, und anschließend
b) aus diesem Bisphenol A enthaltenden Produktgemisch ein Bisphenol A-Phenol-Addukt durch Kristallisation und anschließende Filtration und Wäsche abgetrennt wird,
c) aus dem so erhaltenen BPA-Phenol-Adduktkristallen das Phenol ganz oder teilweise abgetrennt wird
wobei die Kristallisation in Schritt b) in Form einer kontinuierlichen Suspensionskristallisation durchgeführt wird und dabei wenigstens drei Kristallisationsvorrichtungen enthaltend ein Kristallissationsgefäss, einen Wärmeaustauscher und eine Umwälzpumpe, verwendet werden, die derart geschaltet sind, dass das Produktgemisch zunächst in zwei parallel geschalteten Kristallisationsvorrichtungen auf eine Temperatur zwischen 50 und 70 °C und anschließend in einer dritten Kristallisationsvorrichtung, die seriell zu den zwei ersten Kristallisationsvorrichtungen nachgeschaltet ist, auf eine Temperatur zwischen 40 und 50 °C abgekühlt wird, wobei die Gesamtverweilzeit des Produktgemisches in der Kristallisation über alle Kristallisationsvorrichtungen gesehen mehr als 4 h beträgt.

Vorteilhaft ist das erfindungsgemäße Verfahren, wenn die 1. Stufe der Kristallisation mindestens 50 bis 85 % des gesamten Kristallisationsvolumens der zweistufigen Kristallisation umfasst, wobei das Gesamtvolumen der Kristaller mindestens das 4-fache der in die zweistufige Kristallisation geführten produkthaltigen Suspension pro Zeiteinheit beträgt.

Vorteilhaft ist das erfindungsgemäße Verfahren, wenn die 1. Stufe der Kristallisation mindestens 60 bis 80 % des gesamten Kristallisationsvolumens der zweistufigen Kristallisation umfasst, wobei das Gesamtvolumen der Kristaller mindestens das 4-fache der in die zweistufige Kristallisation geführten produkthaltigen Suspension pro Stunde beträgt.

Vorteilhaft ist das erfindungsgemäße Verfahren, wenn die in die Kristallisation geführte produkthaltige Suspension zusammengesetzt ist aus 15 - 40 Gew.-% BPA, 60 - 85 Gew.-% Phenol, 0,5 - 7 Gew.-%, o,p-BPA,: 0 - 5 Gew.-% Trisphenolen, 0 - 7 Gew.-% Indanen/Indenen, 0 - 7 Gew.-% Chromanen/Chromenen, 0 - 5 Gew.-% Wasser, 0 - 8 Gew.-% Aceton, 0 - 5 Gew.-% o,o-BPA und 0 - 5 Gew.-% Isopropenylphenolen bezogen auf das Gesamtgewicht der Suspension.

Vorteilhaft ist das erfindungsgemäße Verfahren, wenn die in Schritt c) beschriebene Wäsche zumindestens teilweise mit Phenol durchgeführt wird, das aus der Herstellung von Polycarbonat nach dem Schmelzeverfahren stammt.

Das erfindungsgemäße Verfahren wird bevorzugt so durchgeführt, dass die zu kristallisierende Flüssigkeit vor der Kristallisation nach Schritt b) auf Temperaturen unterhalb von 80 °C, bevorzugt auf Temperaturen von 75°C, insbesondere 75 bis > 70°C abgekühlt wird.

Die im erfindungsgemäßen Verfahren durchgeführte Kristallisation nach Schritt b) erfolgt in wenigstens drei Kristallisationsvorrichtungen, die jeweils ein Kristallisationsgefäß, eine Umwälzpumpe und einen Wärmeaustauscher enthalten. Bevorzugt weist jede Kristallisationsvorrichtung ein oder mehrere Wärmeaustauscher auf. Besonders bevorzugt weist jede Kristallisationsvorrichtung einen Wärmeaustauscher auf. Das Produktgemisch, das aus der Reaktion von Phenol mit Aceton entstanden ist und aus dem das Bisphenol A-Phenol-Addukt auskristallisiert wird, wird bevorzugt unmittelbar vor der Kristallisation in den Umwälzstrom eingemischt.

Das Gesamtvolumen der insgesamt eingesetzten Kristallisationsvorrichtungen entspricht mindestens dem 4-fachen der in die Kristallisation geführten produkthaltigen Suspensionsvolumen pro Stunde.

Während der Kristallisation nach Schritt b) wird die Kristallisation in zwei Stufen durchgeführt. Hierbei besteht die erste Stufe aus mindestens zwei parallel geschalteten Kristallisationsvorrichtungen. Es können auch noch weitere Kristallisationsvorrichtungen zu diesen beiden in der ersten Stufe dazu geschaltet werden, wobei diese ebenfalls zu den anderen beiden parallel geschaltet sein müssen. Innerhalb der ersten Stufe der Kristallisation nach Schritt b) wird mindestens 50 % des gesamten Kristallisationsvolumen über alle Stufen der Kristallisation nach Schritt b) umgesetzt, bevorzugt 55 %, besonders bevorzugt mindestens 60 %. Die Temperatur innerhalb der ersten Kristallisationsstufe liegt unterhalb von 70°C, bevorzugt unterhalb von 65°C, besonders bevorzugt im Bereich von 50 bis 65 °C, ganz besonders bevorzugt im Bereich von 50 bis 62 °C. Im Zufluss der ersten Kristallisationsstufe beträgt der Bisphenol A-gehalt des Zuflusses 15 bis 40 Gew.-%, bevorzugt 15 bis 35 Gew.%, besonders bevorzugt 20 bis 35 Gew.%. In der ersten Kristallisationsstufe werden mindestens zwei Kristallisationsvorrichtungen enthaltend ein Kristallisationsgefäß, einen Kühler und eine Umwälzpumpe parallel zu einander betrieben. Das bevorzugte Fassungsvolumen dieser beiden Kristallisationsvorrichtungen in der ersten Kristallisationsstufe ist gleich groß. Bevorzugt ist das Fassungsvolumen der Kristallisationsvorrichtungen der ersten Stufe um 20% größer als das der zweiten Stufe, besonders bevorzugt ist das Fassungsvolumen innerhalb der ersten Stufe eineinhalb mal so groß wie das der zweiten Stufe. Ganz besonders bevorzugt sind die Fassungsvolumina aller Kristallisationsvorrichtungen gleich groß.

Zu den beiden ersten Kristallisationsvorrichtungen ist in der zweiten Stufe mindestens eine Kristallisationsvorrichtung seriell zu geschaltet. Die Temperatur in dem Kristallisationsgefäß der zweiten Stufe ist niedriger als in der ersten Stufe, bevorzugt unterhalb von 50°C, besonders bevorzugt unterhalb von 45°C, ganz besonders bevorzugt im Bereich von 40 bis 43°C. Nach Austritt aus der zweiten Kristallisationsstufe, die mindestens ein oder aber mehrere Kristallisationsvorrichtungen enthält, wobei wenigstens eine seriell zu den Kristallisationsvorrichtungen der ersten Stufe geschaltet ist, beträgt der Gehalt an Bisphenol in gelöster Form in der Mutterlauge bei Austritt aus der letzten Kristallisationsvorrichtung der zweiten Stufe 5 bis 20 Gew.-% bezogen auf das Gesamtgewicht der flüssigen Mutterlauge, besonders bevorzugt 5 bis 15 Gew.-%.

Anschließend wird die aus der zweiten Kristallisationsstufe erhaltene Suspension filtriert, wobei ein Bisphenol A-Phenol-Addukt erhalten wird, das durch weitere dem Fachmann bekannte Wäschen gereinigt und anschließend durch weitere dem Fachmann bekannte Trennoperationen wie beispielsweise Verdampfungen, Destillationen oder Strippen in Bisphenol A und Phenol aufgetrennt wird. Durch das erfindungsgemäße Verfahren wird so ein Bisphenol A mit einer Reinheit von größer 99,7% bezogen auf die Gesamtmenge des erhaltenen Produktes unter Ausschluss von noch enthaltenem Phenol erhalten.

Das erfindungsgemäße Verfahren zur Herstellung von Kristallen aus Addukten aus Bisphenol A und Phenol hat den folgenden Vorteil: Es liefert die Addukte aus Bisphenol A und Phenol in einer so hohen Reinheit, dass die üblicherweise notwendigen weiteren Aufarbeitungsschritte zur Herstellung eines für hochwertige Folgeprodukte, beispielsweise Polycarbonate, Epoxydharze, Formaldehydharze u. a. geeigneten Bisphenol As, vermieden werden können. Die genannten üblicherweise notwendigen weiteren Aufarbeitungsschritte sind beispielsweise zusätzliche Kristallisationsschritte oder zusätzliche Destillationsschritte.

Ein weiterer Vorteil des erfindungsgemäßen Verfahrens ist, dass durch die zur Verfügung stehenden höheren Verweilzeiten in der Hochtemperaturstufe die Übersättigung in dieser Kristallisationsstufe sinkt, wodurch die Wärmeaustauscher seltener von anhaftenden, auskristallisierten Ablagerungen gereinigt werden müssen.

Ein weiterer Vorteil des erfindungsgemäßen Verfahrens besteht darin, dass durch die parallele Betriebsweise der Kristaller der ersten Stufe während der Abschmelzung eines Kristallers ein weiterer auf der gleichen Temperaturstufe in Betrieb bleibt, wodurch ein Umstellen auf eine einstufige Kristallisation mit ausschließlicher Niedrigtemperaturstufe vermieden wird. Dadurch kann die Anlagenverfügbarkeit sowie gleichzeitig die Produktreinheit erhöht werden.

Ein weiterer Vorteil des erfindungsgemäßen Verfahrens ist die Aufrechthaltung einer höhere Produktionskapazität, wenn Wärmeaustauscher durch den Produktionsbetrieb bereits teilweise mit anhaftenden, auskristallisierten Ablagerungen belegt sind. Da die teilweise auskristallisierten Ablagerungen im Wärmeaustauscher den Wärmeübergang verschlechtern, muss dies bei konstanter Produktionsleistung durch Absenkung der Kühlwassertemperatur ausgeglichen werden. Dies führt wiederum zu einer beschleunigten Belegung des Wärmeaustauschers mit auskristallisierenden Ablagerungen und somit zu einer häufiger notwendigen Abschmelzung eines Kristallers. Durch das erfindungsgemäße Verfahren kann im Fall teilweise belegter Wärmeaustauscher der Produktionsmengenstrom an den jeweiligen Belegungsgrad der Wärmeaustauscher angepasst werden und so bei gleichzeitiger Aufrechterhaltung der Produktqualität die Produktionsleistung auf hohem Niveau gehalten werden.

Beim erfindungsgemäßen Verfahren zur Herstellung von Kristallen aus dem Addukt aus einem Bisphenol und einem Phenol ist es bevorzugt, dass die Umwälzkühler mit temperiertem Warmwasser als Kühlmedium betrieben werden. Hierbei ist es insbesondere bevorzugt, dass die Temperaturdifferenz zwischen temperiertem Warmwasser und zu kühlender Suspension < 8 K, ganz besonders bevorzugt < 5 K, beträgt.

Beim erfindungsgemäßen Verfahren zur Herstellung von Kristallen aus dem Addukt aus einem Bisphenol und einem Phenol ist es bevorzugt, dass der Kristaller und die zugehörigen Peripheriegeräte in regelmäßigen Zeitabständen von bevorzugt 25 bis 60 Tagen durch Aufheizung auf bevorzugt 80°C oder höher gereinigt werden, wobei der Umwälzkreislauf weiter betrieben wird.

Beim erfindungsgemäßen Verfahren zur Herstellung von Kristallen aus dem Addukt aus Bisphenol und Phenol findet bevorzugt eine Suspensionskristallisation in Form einer Umwälzkristallisation statt.

Nach dem erfindungsgemäßen Verfahren können auch andere Bisphenole als Bisphenol A hergestellt werden.

Für das erfindungsgemäße Verfahren wird unsubstituiertes Phenol eingesetzt. Sollten andere Bisphenole nach dem obigen Verfahren hergestellt werden können auch andere Phenol-Derivate eingesetzt werden.

Gegenstand der Erfindung ist ein Verfahren zur Herstellung von BPA bzw. BPA-Phenol-Adduktkristallen, bei dem ein Gemisch enthaltend folgende Komponenten in die Kristallisation eingespeist wird:
BPA: 15 - 40 Gew.-%, bevorzugt 15 - 35 Gew.-%, besonders bevorzugt 20 - 35 Gew.-%,
Phenol: 60 - 85 Gew.-%, bevorzugt 65 - 85 Gew.-%, besonders bevorzugt 65 - 80 Gew.-%,
o,p-BPA: 0,5 - 7 Gew.-%, bevorzugt 1 - 6 Gew.-%, besonders bevorzugt 1 - 5 Gew.-%,
Trisphenole: 0 - 5 Gew.-%, bevorzugt 0 - 3 Gew.-%
Indane/Indene: 0 - 7 Gew.-%, bevorzugt 0 - 5 Gew.-%
Chromane/Chromene: 0 - 7 Gew.-%, bevorzugt 0 - 5 Gew.-%
Wasser: 0 - 5 Gew.-%, bevorzugt 0 - 3 Gew.-%
Aceton: 0 - 8 Gew.-%, bevorzugt 0 - 5 Gew.-%, besonders bevorzugt 0 - 2 Gew.-%,
o,o-BPA: : 0 - 5 Gew.-%, bevorzugt 0 - 3 Gew.-%
Isopropenylphenole: : 0 - 5 Gew.-%, bevorzugt 0 - 3 Gew.-%
bezogen auf das Gesamtgewicht der Suspension.

Optional kann das Produktgemisch vor der Suspensionskristallisation durch (Vakuum-)Destillation ganz oder teilweise von darin enthaltenem Wasser, nicht umgesetzten Aceton und anderen leichtersiedenden Verbindungen befreit werden.

Durch den Einsatz von zur Abtrennung von Feststoffen geeigneten Filtermedien werden die Mischkristalle Bisphenol A-Phenol (Bisphenol A-Phenol-Addukt) von der Mutterlauge abgetrennt. Bevorzugt ist in oder auf diesen Apparaten auch eine Wäsche des abgetrennten Feststoffes möglich. Dies kann beispielsweise auf entsprechenden Zentrifugen wie Schälzentrifugen, Siebschneckenzentrifugen oder Schubzentrifugen, aber auch auf Drehfiltern, Bandfiltern und (Vakuum-)Scheibenfiltern erreicht werden. Bevorzugt kommen Druckdrehfilter, besonders bevorzugt Vakuumdrehfilter, zum Einsatz.

Die Wäsche des abgetrennten Feststoffes (Filterkuchen) kann auch im Anschluss der Fest/flüssig-Trennung in einer geeigneten Apparatur separat durchgeführt werden.

Der Filterkuchen aus den Bisphenol A-Phenol-Mischkristallen wird mit frischem Phenol und / oder rückgeführtem Phenol aus dem Herstellungsprozess für Bisphenol A, das ggf. vorher destillativ aufgereinigt wurde, gewaschen. Die Wäsche wird bevorzugt bei einer Temperatur des Phenols von 40-70°C durchgeführt.

Zur Wäsche kann auch Phenol verwendet werden, das bei der Herstellung von Polycarbonat nach dem Schmelzeprozess anfällt.

Die gewaschenen Mischkristalle werden dann vorzugsweise aufgeschmolzen und die erhaltene Schmelze wie aus dem Stand der Technik bekannt, vom Phenol befreit. Bevorzugt wird die Schmelze in einer zweistufigen Fahrweise von Phenol und anderen leichtflüchtigen Verunreinigungen befreit. Dabei wird die Schmelze im ersten Schritt mit sowohl einem Vakuum als auch erhöhter Temperatur und im zweiten Schritt mit einem Desorptionsgas, bevorzugt Stickstoff, bei Temperaturen zwischen 160 - 210°C behandelt, wobei Phenol bis auf Restgehalte von < 200 ppm entfernt wird.

In einer anderen bevorzugten Ausführungsform erfolgt die Phenolentfernung nur einstufig bei erhöhter Temperatur und im Vakuum, wobei Restgehalte an Phenol im Bisphenol von bis zu 10 Gew.-% bezogen auf die Gesamtmenge an Produkt eingestellt werden können.

Bisphenol A, das nach dem erfindungsgemäßen Verfahren hergestellt wurde, kann mit Phosgen nach dem Phasengrenzflächenverfahren oder mit Diarylcarbonaten, bevorzugt Diphenylcarbonat, nach dem Schmelzeverfahren zu Polycarbonat umgesetzt werden. Das nach dem erfindungsgemäßen Verfahren hergestellte BPA kann weiterhin als Ausgangsstoff zur Herstellung verschiedener polymerer Werkstoffe wie beispielsweise Polyarylate, Polyetherimide, modifizierte Phenol-Formaldehydharze und Epoxyharze dienen.

Im Folgenden wird die Erfindung anhand von Beispielen erläutert, ohne auf diese beschränkt zu sein.

### Beispiele

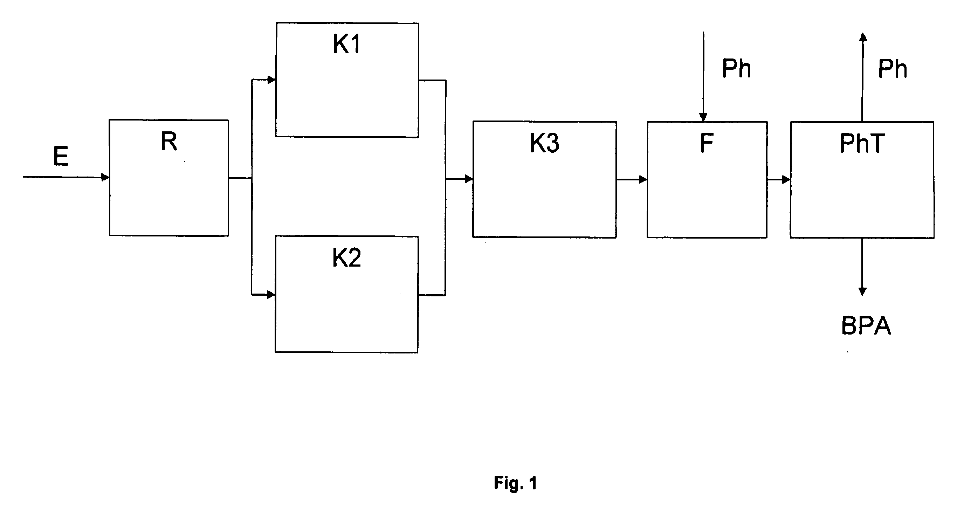

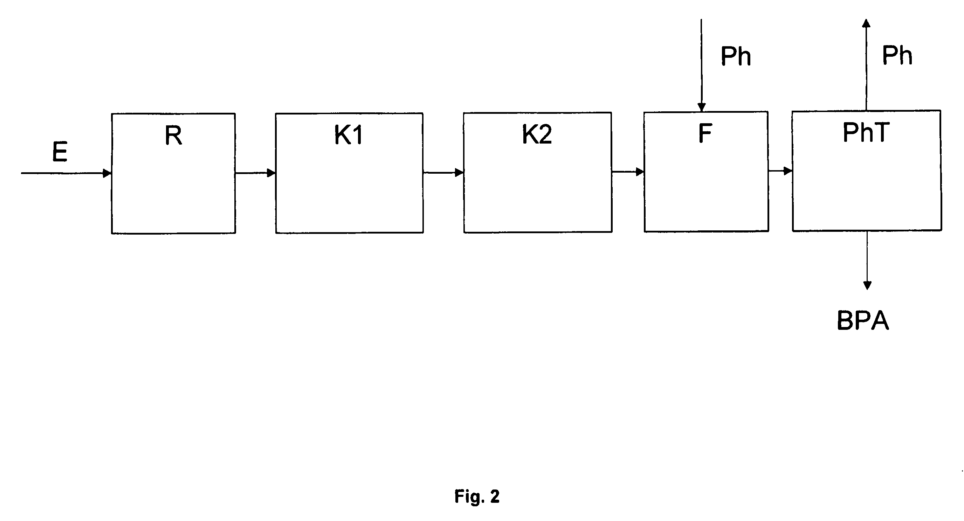

Fig. 1 beschreibt schematisch eine bevorzugte Ausführungsform des erfindungsgemäßen Verfahrens, worin drei Kristallisationsvorrichtungen (K1, K2 und K3) verwendet werden, von denen die ersten beiden (K1 und K2) parallel betrieben und die dritte (K3) zu diesen beiden jeweils in Serie geschaltet ist. Fig. 2 beschreibt schematisch ein Verfahren, worin lediglich zwei Kristallisationsvorrichtungen (K1 und K2) verwendet werden, die in Serie geschaltet sind. Darin bedeuten
- R: Reaktionsgefäß bzw. Reaktoranordnung, in dem die Bisphenol A-Herstellung durchgeführt wird
- E: Feed zur Bisphenol A-Herstellung
- K1: Kristallisationvorrichtung 1 (kurz auch als Kristaller 1 bezeichnet)
- K2: Kristallisationvorrichtung 2 (kurz auch als Kristaller 2 bezeichnet)
- K3: Kristallisationvorrichtung 3 (kurz auch als Kristaller 3 bezeichnet)
- F: Filtration
- PhT: Phenolabtrennung
- Ph: Phenol
- BPA: Bisphenol A

### Beispiel 1 (erfindungsgemäß)

Zur Aufarbeitung und Reinigung von BPA wurde die Reaktionsmischung aus der BPA-Herstellung R in Fig. 1 einer Suspensionskristallisation in einer Vorrichtungsanordnung aus drei Kristallisationsvorrichtungen (K1, K2 und K3 gemäß Fig. 1) zugeführt. Hierzu wurde eine 2-stufige Kristallisation, beginnend in der 1. Stufe bei 54 °C und der 2. Stufe bei 41°C durchgeführt, wobei die 1. Stufe aus zwei parallel betriebenen Kristallisationsvorrichtungen (K1 und K2), die 2. Stufe aus einer Kristallisationsvorrichtung (K3), dessen Volumen 80 % der Summe der Kristallisationsvorichtungen K1 und K2 der 1. Stufe betrug, bestand. Der Bisphenol A-Gehalt der zu kristallisierenden Reaktionslösung betrug 30 %. Die Reaktionsmischung wurde unmittelbar vor den Kristallisationsvorrichtungen K1 und K2 mit 70°C in den Umwälzstrom eingemischt.

Die Umwälzkühler wurden von oben nach unten geführt und die Kühler mit temperiertem Warmwasser betrieben. Die maximale Temperaturdifferenz zwischen temperiertem Warmwasser und zu kühlender Suspension betrug 3 K.

Die fertige Mischkristallsuspension wurde nach einer Gesamtverweilzeit in allen Kristallern von 8,5 Stunden dem Kristaller K3 entnommen.

Für die Durchführung wurden als Wärmetauscher Rohrbündelwärmetauscher eingesetzt. Eine durch Ablagerungen an Innenflächen der Kühlrohre bedingte Beeinträchtigung des Wärmeübergangs konnte innerhalb eines Zeitraums von 3 Monaten nicht festgestellt werden, so dass während dieses Zeitraums die Innenflächen der Kühlrohre nicht von BPA- und BPA/Phenol-Belägen befreit werden mussten.

Durch diese Art der Kristallisation konnten Bisphenol A/Phenol-Adduktkristalle mit hohen Reinheiten erhalten werden.

Nach Filtration F und Phenolabtrennung PhT wurde Bisphenol A (BPA) mit einer Reinheit von 99,75 % und einer Farbzahl von ca. 15 Hazen. erhalten. Die Hazen-Farbzahl wurde visuell durch den Vergleich mit APHA Standard-Colormetrie-Lösungen gemäß ASTM D 1209-97 bestimmt.

### Beispiel 2 (erfindungsgemäß)

Zur Aufarbeitung und Reinigung von BPA wurde die Reaktionsmischung aus der BPA-Herstellung R in Fig. 1 einer Suspensionskristallisation in einer Vorrichtungsanordnung aus drei Kristallisationsvorrichtungen (K1, K2 und K3 gemäß Fig. 1) zugeführt. Hierzu wurde eine 2-stufige Kristallisation, beginnend in der 1. Stufe bei 54 °C und der 2. Stufe bei 41°C durchgeführt, wobei die 1. Stufe aus zwei parallel betriebenen Kristallisationsvorrichtungen (K1 und K2), die 2. Stufe aus einer Kristallisationsvorrichtung (K3), dessen Volumen 50 % der Summe der Kristallisationsvorichtungen K1 und K2 der 1. Stufe betrug, bestand. Der Bisphenol A-Gehalt der zu kristallisierenden Reaktionslösung betrug 30 %. Die Reaktionsmischung wurde unmittelbar vor den Kristallisationsvorrichtungen K1 und K2 mit 70°C in den Umwälzstrom eingemischt.

Der Umwälzstrom der Umwälzkristallisation wurde dem jeweiligen Kristallisationsgefäß am Fuß tangential zugeführt und mittig am Kopf des jeweiligen Kristallisationsgefäßes entnommen.

Die Umwälzkühler wurden von oben nach unten geführt und die Kühler mit temperiertem Warmwasser betrieben. Die maximale Temperaturdifferenz zwischen temperiertem Warmwasser und zu kühlender Suspension betrug 3 K.

Die fertige Mischkristallsuspension wurde nach einer Gesamtverweilzeit in allen Kristallern von 8,5 Stunden dem Kristaller K3 entnommen.

Für die Durchführung wurden als Wärmetauscher Rohrbündelwärmetauscher eingesetzt. Eine durch Ablagerungen an Innenflächen der Kühlrohre der Wärmetauscher bedingte Beeinträchtigung des Wärmeübergangs konnte innerhalb eines Zeitraums von 10 Monaten nicht festgestellt werden, so dass während dieses Zeitraums die Innenflächen der Kühlrohre nicht von BPA- und BPA/Phenol-Belägen befreit werden mussten.

Nach Filtration F und Phenolabtrennung PhT wurde Bisphenol A (BPA) mit einer Reinheit von 99,79 % und einer Farbzahl von < 15 Hazen erhalten. Die Hazen-Farbzahl wurde visuell durch den Vergleich mit APHA Standard-Colormetrie-Lösungen gemäß ASTM D 1209-97 bestimmt.

Durch diese erfindungsgemäße Art der Kristallisation konnten Bisphenol A/Phenol-Adduktkristalle mit hohen Reinheiten erhalten werden. Durch die Wahl des Volumens der Kristallisationsvorrichtung in der zweiten Stufe konnte die Reinheit sogar gegenüber Beispiel 1 noch gesteigert werden.

### Beispiel 3 (Vergleichsbeispiel)

Zur Aufarbeitung und Reinigung von BPA wurde die Reaktionsmischung aus der BPA-Herstellung R in Fig. 2 einer Suspensionskristallisation in einer Vorrichtungsanordnung aus zwei Kristallisationsvorrichtungen (K1 und K2 gemäß Fig. 2) zugeführt. Hierzu wurde eine 2-stufige Kristallisation, beginnend in der 1. Stufe (K1) bei 54 °C und der 2. Stufe (K2) bei 41°C durchgeführt, wobei beide Stufen in den Kristallisationsvorrichtungen (K1 und K2) das gleiche Kristallisationsvolumen aufwiesen. Der Bisphenol A-Gehalt der zu kristallisierenden Reaktionslösung betrug 30 %. Die Reaktionsmischung wurde unmittelbar vor der Kristallisationsvorrichtung K1 mit 70°C in den Umwälzstrom eingemischt.

Der Umwälzstrom der Umwälzkristallisation wurde dem jeweiligen Kristallisationsgefäß am Fuß tangential zugeführt und mittig am Kopf des jeweiligen Kristallisationsgefäßes entnommen.

Die Umwälzkühler wurden von oben nach unten und die Kühler mit temperiertem Warmwasser betrieben. Die maximale Temperaturdifferenz zwischen temperiertem Warmwasser und zu kühlender Suspension betrug 3 K.

Die fertige Mischkristallsuspension wurde nach einer Gesamtverweilzeit in allen Kristallern von 8,5 Stunden dem K2 entnommen.

Für die Durchführung wurden als Wärmetauscher Rohrbündelwärmetauscher eingesetzt. Zur Befreiung von BPA- und BPA/Phenol-Belägen mussten die Innenflächen der Kühlrohre in ca. 2-monatlichen Zeitabständen durch eine Schnellaufheizung auf 80°C gereinigt bzw. das gesamte Kristallisationsvorrichtungssystem durch Aufheizung des Produktinhaltes und Weiterbetrieb des Umwälzkreislaufes gereinigt werden.

Nach Filtration F und Phenolabtrennung PhT von den so erhaltenen Bisphenol A/Phenol-Adduktkristallen wurde Bisphenol A (BPA) mit einer Reinheit von 99,72 % und einer Farbzahl von 20 Hazen erhalten. Die Hazen-Farbzahl wurde visuell durch den Vergleich mit APHA Standard-Colormetrie-Lösungen gemäß ASTM D 1209-97 bestimmt.

## Patentansprüche

1. Verfahren zur Herstellung von Bisphenol A mit einer Reinheit von mehr als 99,7%, bei dem
a) Phenol und Aceton in Gegenwart eines sauren Katalysators zu einem Bisphenol A enthaltenden Produktgemisch umgesetzt werden, und anschließend
b) aus diesem Bisphenol A enthaltenden Produktgemisch ein Bisphenol A-Phenol-Addukt durch Kristallisation und anschließende Filtration und Wäsche abgetrennt wird,
c) aus dem so erhaltenen BPA-Phenol-Adduktkristallen das Phenol ganz oder teilweise abgetrennt wird
wobei die Kristallisation in Schritt b) in Form einer kontinuierlichen Suspensionskristallisation durchgeführt wird und dabei wenigstens drei Kristallisationsvorrichtungen, enthaltend ein Kristallisationsgefäß, einen Wärmeaustauscher und eine Umwälzpumpe, verwendet werden, die derart geschaltet sind, dass das Produktgemisch zunächst in zwei parallel geschalteten Kristallisationsvorrichtungen auf eine Temperatur zwischen 50 und 70 °C und anschließend in einer dritten Kristallisationsvorrichtung, die seriell zu den zwei ersten Kristallisationsvorrichtungen nachgeschaltet ist, auf eine Temperatur zwischen 40 und 50 °C abgekühlt wird, wobei die Gesamtvervveilzeit des Produktgemisches in der Kristallisation über alle Kristallisationsvorrichtungen gesehen mehr als 4 h beträgt.

2. Verfahren nach Anspruch 1 **dadurch gekennzeichnet, dass** die 1. Stufe der Kristallisation mindestens 50 bis 85 % des gesamten Kristallisationsvolumens der zweistufigen Kristallisation umfasst, wobei das Gesamtvolumen der Kristallisationsvorrichtungen mindestens das 4-fache der in die zweistufige Kristallisation geführten produkthaltigen Suspension pro Zeiteinheit beträgt.

3. Verfahren nach Anspruch 1 **dadurch gekennzeichnet, dass** die 1. Stufe der Kristallisation mindestens 60 bis 80 % des gesamten Kristallisationsvolumens der zweistufigen Kristallisation umfasst, wobei das Gesamtvolumen der Kristallisationsvorrichtungen mindestens das 4-fache der in die zweistufige Kristallisation geführten produkthaltigen Suspension pro Stunde beträgt.

4. Verfahren nach Anspruch 1 **dadurch gekennzeichnet, dass** die in die Kristallisation geführte produkthaltige Suspension zusammengesetzt ist aus 15 - 40 Gew.% BPA, 60 - 85 Gew.-% Phenol, 0,5 - 7 Gew.%, o,p-BPA, 0 - 5 Gew.% Trisphenolen, 0 - 7 Gew.-% Indanen/Indenen, 0 - 7 Gew.-% Chromanen / Chromenen, 0 - 5 Gew.% Wasser, 0 - 8 Gew.% Aceton, 0 - 5 Gew.-% o,o-BPA und 0 - 5 Gew.-% Isopropenylphenolen bezogen auf das Gesamtgewicht der Suspension.

5. Verfahren nach Anspruch 1 **dadurch gekennzeichnet, dass** die in Schritt c) beschriebene Wäsche zumindest teilweise mit Phenol durchgeführt wird, das aus der Herstellung von Polycarbonat nach dem Schmelzeverfahren stammt.

## Claims

1. Process for preparing bisphenol A with a purity of more than 99.7%, in which
a) phenol and acetone are converted in the presence of an acidic catalyst to give a product mixture comprising bisphenol A, and then
b) a bisphenol A-phenol adduct is removed from this product mixture comprising bisphenol A by crystallization and subsequent filtration and washing,
c) the phenol is completely or partially removed from the BPA-phenol adduct crystals thus obtained,
the crystallization in step b) being performed in the form of a continuous suspension crystallization using at least three crystallization apparatuses comprising a crystallization vessel, a heat exchanger and a circulation pump which are connected such that the product mixture is first cooled to a temperature between 50 and 70 °C in two parallel crystallization apparatuses and then to a temperature between 40 and 50 °C in a third crystallization apparatus which is connected in series with respect to the first two crystallization apparatuses, the total residence time of product mixture in the crystallization viewed over all crystallization apparatuses being more than 4 h.

2. Process according to Claim 1, **characterized in that** the 1^{st} stage of the crystallization comprises at least 50 to 85% of the total crystallization volume of the two-stage crystallization, the total volume of the crystallization apparatuses being at least 4 times the product-containing suspension conducted into the two-stage crystallization per unit time.

3. Process according to Claim 1, **characterized in that** the 1^{st} stage of the crystallization comprises at least 60 to 80% of the total crystallization volume of the two-stage crystallization, the total volume of the crystallization apparatuses being at least 4 times the product-containing suspension conducted into the two-stage crystallization per hour.

4. Process according to Claim 1, **characterized in that** the product-containing suspension conducted into the crystallization is composed of 15-40% by weight of BPA, 60-85% by weight of phenol, 0.5-7% by weight of o,p-BPA, 0-5% by weight of trisphenols, 0-7% by weight of indanes/indenes, 0-7% by weight of chromanes/chromenes, 0-5% by weight of water, 0-8% by weight of acetone, 0-5% by weight of o,o-BPA and 0-5% by weight of isopropenylphenols, based on the total weight of the suspension.

5. Process according to Claim 1, **characterized in that** the washing described in step c) is performed at least partly with phenol which originates from the preparation of polycarbonate by the melt process.

## Revendications

1. Procédé pour la préparation de bisphénol A présentant une pureté de plus de 99,7%, dans lequel
a) on transforme du phénol et de l'acétone en présence d'un catalyseur acide en un mélange de produits contenant du bisphénol A, puis
b) on sépare de ce mélange de produits contenant du bisphénol A, un produit d'addition bisphénol A-phénol par cristallisation et filtration et lavage consécutifs,
c) on sépare totalement ou partiellement le phénol des cristaux de produits d'addition BPA-phénol ainsi obtenus
où la cristallisation dans l'étape b) est réalisée sous forme d'une cristallisation continue en suspension et on y utilise au moins trois dispositifs de cristallisation, contenant un récipient de cristallisation, un échangeur thermique et une pompe de circulation, qui sont commutés de manière telle que le mélange de produits est d'abord refroidi dans deux dispositifs de cristallisation commutés en parallèle à une température entre 50 et 70°C, puis dans un troisième dispositif de cristallisation, qui est disposé en aval et commuté en série par rapport aux deux premiers dispositifs de cristallisation, à une température entre 40 et 50°C, le temps de séjour total du mélange de produits dans la cristallisation, pris en considération sur l'ensemble des dispositifs de cristallisation, étant supérieur à 4 h.

2. Procédé selon la revendication 1, **caractérisé en ce que** la première étape de la cristallisation représente au moins 50 à 85% du volume de cristallisation total de la cristallisation en deux étapes, où le volume total des dispositifs de cristallisation représente au moins 4 fois la suspension contenant du produit alimentée dans la cristallisation en deux étapes par unité de temps.

3. Procédé selon la revendication 1, **caractérisé en ce que** la première étape de la cristallisation représente au moins 60 à 80% du volume de cristallisation total de la cristallisation en deux étapes, où le volume total des dispositifs de cristallisation représente au moins 4 fois la suspension contenant du produit alimentée dans la cristallisation en deux étapes par heure.

4. Procédé selon la revendication 1, **caractérisé en ce que** la suspension contenant des produits guidée dans la cristallisation est composée de 15-40% en poids de BPA, 60-85% en poids de phénol, 0,5-7% en poids d'o,p-BPA, 0-5% en poids de trisphénols, 0-7% en poids d'indanes/indènes, 0-7% en poids de chromanes/chromènes, 0-5% en poids d'eau, 0-8% en poids d'acétone, 0-5% en poids d'o,o-BPA et 0-5% en poids d'isopropénylphénols, par rapport au poids total de la suspension.

5. Procédé selon la revendication 1, **caractérisé en ce que** le lavage décrit dans l'étape c) est réalisé au moins partiellement avec du phénol, qui provient de la préparation de polycarbonate selon le procédé en masse fondue.
